# EUROPEAN PATENT SPECIFICATION

(11) **EP 2 449 383 B1**
(45) Date of publication and mention of the grant of the patent: **16.05.2018**
(21) Application number: 10730098.0
(22) Date of filing: 02.07.2010
(51) Int. Cl.: G01N 33/574

(54) **DIAGNOSTIC METHOD FOR PREDICTING THE RISK OF CANCER RECURRENCE BASED ON HISTONE MACROH2A ISOFORMS**
DIAGNOSEVERFAHREN ZUR VORHERSAGE DES KREBSREKURRENZRISIKOS BASIEREND AUF HISTON-MACROH2A-ISOFORMEN
PROCÉDÉ DE DIAGNOSTIC POUR PRÉDIRE LE RISQUE DE RÉCURRENCE DE CANCER BASÉ SUR DES ISOFORMES MACROH2A D'HISTONE

(30) Priority: 02.07.2009 EP 09008679
(43) Date of publication of application: 09.05.2012
(73) Proprietor: European Molecular Biology Laboratory (EMBL), 69117 Heidelberg (DE); Thoraxklinik Heidelberg GmbH, 69126 Heidelberg (DE)
(72) Inventor: LADURNER, Andreas, 69151 Neckargemünd (DE); SPORN, Judith, Chicago IL 60611 (US); MULEY, Thomas, 69509 Mörlenbach (DE)
(74) Representative: Krauss, Jan
(86) International application number: PCT/EP2010/004008
(87) International publication number: WO 2011/000573

(56) References cited:
- CHANGOLKAR LAKSHMI N ET AL: "Developmental changes in histone macroH2A1-mediated gene regulation" MOLECULAR AND CELLULAR BIOLOGY, vol. 27, no. 7, April 2007 (2007-04), pages 2758-2764, XP002547973 ISSN: 0270-7306
- PEHRSON JOHN R ET AL: "Developmental and tissue expression patterns of histone macroH2A1 subtypes" JOURNAL OF CELLULAR BIOCHEMISTRY, vol. 65, no. 1, 1997, pages 107-113, XP002547974 ISSN: 0730-2312
- SPORN J C ET AL: "Histone macroH2A isoforms predict the risk of lung cancer recurrence" ONCOGENE, no. 28, 3 August 2009 (2009-08-03), pages 3423-3428, XP002547975
- COLLADO M ET AL: "The power and the promise of oncogene-induced senescence markers" NATURE REVIEWS CANCER 200606 GB, vol. 6, no. 6, June 2006 (2006-06), pages 472-476, XP002547976 ISSN: 1474-175X
- ROEPMAN P ET AL: "An immune response enriched 72-gene prognostic profile for early-stage non-small-cell lung cancer" CLINICAL CANCER RESEARCH 20090101 US, vol. 15, no. 1, 1 January 2009 (2009-01-01), pages 284-290, XP002547977 ISSN: 1078-0432
- GEIGL JOCHEN B ET AL: "Analysis of gene expression patterns and chromosomal changes associated with aging.", CANCER RESEARCH 01 DEC 2004, vol. 64, no. 23, 1 December 2004 (2004-12-01), pages 8550-8557, ISSN: 0008-5472
- WU NAN ET AL: "Histone H2A has a novel variant in fish oocytes.", BIOLOGY OF REPRODUCTION AUG 2009, vol. 81, no. 2, August 2009 (2009-08), pages 275-283, ISSN: 1529-7268

## Description

The present invention relates to an *in vitro* method for diagnosing the risk of cancer recurrence in a mammalian patient, comprising detecting the expression of macroH2A1.1 and/or macroH2A2 in a biological sample obtained from said patient, wherein a low or reduced expression of said macroH2A1.1 and/or macroH2A2 is indicative for an increased risk of cancer recurrence in said patient.

### Background of the invention

Among cancers, lung cancer is the second most common cancer regarding new cases (15% of all cancers in the US) (Jemal et al., 2007) and is the leading cause of cancer deaths worldwide (31% in men and 26% women in the US). Four out of five lung carcinomas are non-small cell lung cancers (NSCLC) and about one third of NSCLC patients are diagnosed with an early disease. Surgery is the best treatment option for these patients, yet a high percentage of patients relapse and: the majority die of their disease. Patients within identical cancer stages often show differing recurrence rates (Zhu et al., 2006); therefore there is an interest in molecular markers, which could identify patients more likely to recur after surgery (D'Amico, 2008). Several potential markers for metastatic recurrence of NSCLC have been tested, but none of them has proven sufficiently useful (Zhu et al., 2006).

Histones are often assumed to be present at similar levels in distinct cell types. The heterochromatic histone variant macroH2A is unusual in several molecular and cellular features. At the structural level, it bears a large C-terminal extension, the macro domain, a globular module of approx. 25 kDa, which in the case of macroH2A1.1 binds ADP-ribose with high specificity (Kustatscher el al., 2005). Further, there are two genes for macro-H2A, conserved in all vertebrates, and a second splice variant for macroH2A1, known as macroH2A1.2, which cannot bind small molecules.

MacroH2A has been implicated in a permanent growth-arrest known as senescence (Pehrson and Fried, 1992; Zhang et al 2005). This phenomenon has been described-for-primary cell cultures *in vitro* (Hayflick, 1965) and is a response to stresses like telomere shortening, accumulation of DNA damage and the overexpression of certain oncogenes (Serrano et al., 1991). It is furthermore interesting to note that oncogene-induced senescence has emerged as a potent antitumor mechanism *in vivo* (Braig et al., 2005; Chen et al., 2005; Collado et al., 2005; Michaloglou et al., 2005).

Holdenrieder et al. (in: Holdenrieder S, Stieber P, Bodenmüller H, Busch M, Fertig G, Fürst H, Schalhorn A, Schmeller N, Untch M, Seidel D. Nucleosomes in serum of patients with benign and malignant diseases. Int J Cancer 2001 Mar 20;95(2):114-20.) observed nucleosomes and the kinetics of the concentration of nucleosomes in serum samples from 20 patients undergoing chemotherapy and from 16 patients undergoing radiotherapy. Sera of patients with malignant tumors contained considerably higher concentrations of nucleosomes compared with those of healthy persons and patients with benign diseases. They propose that the concentration of nucleosomes in serum might be a useful tool for monitoring the biochemical response during antitumor therapy, especially for the early estimation of therapeutic efficacy.

Kurdistani (in: Kurdistani SK. Histone modifications as markers of cancer prognosis: a cellular view. Br J Cancer. 2007 Jul 2;97(1):1-5. Epub 2007 Jun 26.) describes that alterations in modifications of histones have been linked to deregulated expression of many genes with important roles in cancer development and progression. The effects of these alterations have so far been interpreted from a promoter-specific viewpoint, focussing on gene-gene differences in patterns of histone modifications. Furthermore, it is suggested that cancer tissues also display cell-cell differences in total amount of specific histone modifications. This novel cellular epigenetic heterogeneity is related to clinical outcome of cancer patients and may serve as a valuable marker of prognosis.

Barlési et al. (in: Barlési F, Giaccone G, Gallegos-Ruiz MI, Loundou A, Span SW, Lefesvre P, Kruyt FA, Rodriguez JA. Global histone modifications predict prognosis of resected non small-cell lung cancer. J Clin Oncol. 2007 Oct 1;25(28):4358-64.) describe a study examining whether epigenetic modifications may contribute to the development and progression of cancer. Epigenetic changes involving multiple histones were examined for influencing the prognosis of non-small-cell lung cancer (NSCLC) patients. Histone 3 lysine 4 dimethylation (H3K4diMe), and acetylation of histone 2A lysine 5 (H2AK5Ac), histone 2B lysine 12, histone 3 lysine 9 (H3K9Ac), and histone 4 lysine 8 in resected tumor samples of 138 NSCLC patients were examined. Groups determined by stage I patients (below the first node) displayed dramatic differences in survival (median, 10 months in adenocarcinoma patients with H3K9Ac 68% v 147 months in nonadenocarcinoma patients with H3K4diMe 85%). The data as disclosed were said to provide a rationale for the use of a combination of standard chemotherapy with drugs interacting with histone modifications, such as histone deacetylase inhibitors.

Deligezer et al. (in: Deligezer U, Akisik EE, Erten N, Dalay N. Sequence-specific histone methylation is detectable on circulating nucleosomes in plasma. Clin Chem. 2008 Jul;54(7):1125-31. Epub 2008 May 16.) describe that alterations in DNA methylation and histone modifications have been implicated in carcinogenesis and investigated whether histone methylation, as a model of histone modifications, is detectable in plasma. Therefore, monomethylated H3K9 (H3K9me1) was analyzed in plasma by chromatin immunoprecipitation. Further validation was required to prove cancer-specific alterations in histone modifications in the circulation.

Finally, Seligson et al. (in: Seligson DB, Horvath S, McBrian MA, Mah V, Yu H, Tze S, Wang Q, Chia D, Goodglick L, Kurdistani SK. Global Levels of Histone Modifications Predict Prognosis in Different Cancers. Am J Pathol. 2009 Apr 6.) disclose that cancer cells exhibit alterations in histone modification patterns at individual genes and globally at the level of single nuclei in individual cells. Lower global/cellular levels of histone H3 lysine 4 dimethylation (H3K4me2) and H3K18 acetylation (ac) are disclosed to predict a higher risk of prostate cancer recurrence, and it was shown that the cellular levels of both H3K4me2 and H3K18ac also predict clinical outcome in both lung and kidney cancer patients, with lower levels predicting significantly poorer survival probabilities in both cancer groups. Theyalso show that lower cellular levels of H3K9me2, a modification associated with both gene activity and repression, is also prognostic of poorer outcome for individuals with either prostate or kidney cancers. The predictive power of these histone modifications was independent of the proliferation marker Ki-67. Chromatin immunoprecipitation experiments indicated that the lower cellular levels of histone modifications in more aggressive cancer cell lines correlated with lower levels of modifications at DNA repetitive elements but not with gene promoters across the genome. The results suggested that lower global levels of histone modifications are predictive of a more aggressive cancer phenotype, revealing a surprising commonality in prognostic epigenetic patterns of adenocarcinomas of different tissue origins. CHANGOLKAR LAKSHMI N ET AL ("Developmental changes in histone macroH2A1-mediated gene regulation", MOLECULAR AND CELLULAR BIOLOGY, (200704), vol. 27, no. 7, ISSN 0270-7306, pages 2758 - 2764) and PEHRSON JOHN R ET AL ("Developmental and tissue expression patterns of histone macroH2A1 subtypes", JOURNAL OF CELLULAR BIOCHEMISTRY, (1997), vol. 65, no. 1, ISSN 0730-2312, pages 107 - 113) disclose antibodies specific to variants macroH22A1.1, 1.2 and 2.

Lung cancer is the leading cause of cancer deaths. Despite optimal diagnosis and early treatment, many patients die of recurrent disease. There are no sufficiently useful biomarkers to predict the risk of tumor recurrence in cancer, in particular solid cancers, such as lung cancer. It is therefore an object of the present invention, to provide a new diagnostic assay for such recurrent cancer, together with suitable tools for performing said assay. Further objects and advantages will become apparent to the person of skill when reading the following more detailed description of the present invention.

In a preferred first aspect of the present invention, the invention relates to an *in vitro* method for diagnosing the risk of cancer recurrence in a mammalian patient, comprising detecting the expression of macroH2A1.1 and/or macroH2A2 in a biological sample obtained from said patient, wherein a low or reduced expression of said macroH2A1.1 and/or macroH2A2 is indicative for an increased risk of lung cancer recurrence in said patient. Preferably, the method according to the present invention further comprises a prediction of said cancer recurrence based on said diagnosis, further preferred a metastatic recurrence.

In a preferred second aspect of the present invention, the invention relates to an *in-vitro* method for diagnosing the risk of cancer recurrence in a mammalian patient as above, which further comprising a diagnosis of senescent cells, in particular of cancer cells, in the tumor based on the expression of macroH2A1.1.

The present invention is generally based on the finding that expression of histone macroH2A1.1 and macroH2A2 predicts lung cancer recurrence, identifying these histone variants as a novel tool for an improved risk stratification of cancer patients. MacroH2A isoforms are upregulated and highly expressed in cells undergoing senescence, a known anti-tumor mechanism, suggesting macro-H2A1.1 as a useful biomarker for senescent cells in tumors. MacroH2A1.1 is more highly expressed in tumors with better prognosis.

Furthermore, it was found in the context of the present invention that the prognostic correlation of macroH2A1.1 expression is superior to the proliferation marker Ki-67, which is commonly used to estimate cancer patient prognosis. The inventors observe a strong negative correlation between the macroH2A1.1 and macroH2A2 expression and tumor proliferation rate. Slowly proliferating tumors tend to show high expression of macroH2A1.1 and macroH2A2, whereas tumors with a high proliferative index, which are often clinically more aggressive, have lower or reduced or even no expression.

Thus, in a first aspect thereof, the present invention relates to an *in-vitro* method for diagnosing the risk of cancer recurrence in a mammalian patient, comprising detecting the expression of macroH2A1.1 and/or macroH2A2 in a biological sample obtained from said patient, wherein a low or reduced expression of said macroH2A1.1 and/or macroH2A2 is indicative for an increased risk of lung cancer recurrence in said patient. Preferably, the result(s) of the expression analysis can be displayed on a suitable device. In the context of the present invention, a "low" or "reduced" expression of macroH2A1.1 and/or macroH2A2 shall mean that compared to a sample from the same patient before the relapse or a different patient that does not suffer from a risk for relapse, or a healthy patient or group of patients, the expression of macroH2A1.1 and/or macroH2A2 is reduced. Another strategy to determine a low or reduced expression is a lack of staining on histological slides using, preferably, a macroH2A1.1 and/or macroH2A2-specific antibody, as described further below. Yet another strategy is to compare the expression macroH2A1.1 and/or macroH2A2 with the expression of macroH2A1.2 in the sample(s), and in a preferred embodiment of the method according to the present invention, the expression is normalized to the expression of macroH2A1.2 in said sample and/or a control sample. Again, the control sample can be a sample from the same patient before the relapse or a different patient that does not suffer from a risk for relapse, or a healthy patient or group of patients.

In a preferred embodiment thereof, the present invention relates to an *in-vitro* method for diagnosing the risk of cancer recurrence in a mammalian patient, comprising detecting the expression of macroH2A1.1 and/or macroH2A2 in a biological sample obtained from said patient, and comparing said expression of macroH2A1.1 and/or macroH2A2 to a control sample, wherein a low or reduced expression of said macroH2A1.1 and/or macroH2A2 is indicative for an increased risk of lung cancer recurrence in said patient. Preferably, the result(s) of the expression analysis and/or comparison can be displayed or put out on a suitable device, such as a diagnostic computer.

In a further preferred embodiment of the method according to the present invention, the correlation between the expression of macroH2A1.1 or macroH2A2 and the disease-free survival has a significance of *P* < 0.05, preferably *P* < 0.01, and more preferably *P* < 0.006.

A further preferred embodiment of the method according to the present invention, further comprises detecting the expression of additional cancer markers, such as Ki-67. Preferably, the expression of said macroH2A1.1 and/or macroH2A2 in said biological sample inversely correlates with the expression of Ki-67.

Yet another preferred aspect of the method according to the present invention further comprises the step of a prediction of said cancer recurrence based on said diagnosis according to the present invention. "Recurrence" as used herein refers to a clinical manifestation of parameters of the cancer(s) after a disease-free survival time of the patient, such as, preferably, the occurrence of tumor tissue and metastases. The prediction (or likelihood) can also include additional parameters, such as, for example, clinical parameters of the patient or patient group, the type of tumor, etc. The result of the prediction can preferably be displayed on a suitable device.

The method according to the present invention can be used to diagnose any type of solid cancer, Thus, preferred is a method according to the present invention, wherein said cancer is selected from the group of solid cancer, lung cancer, breast cancer, kidney cancer, liver cancer, head and neck cancer, prostate cancer, pancreatic cancer, stomach cancer, colon cancer, thyroid cancer, esophageal cancer, and brain cancer. More preferred is the method for the diagnosis for breast cancer or lung cancer.

Any biological sample can be used for the method according to the present invention, as long as it allows for a direct or indirect detection of the expression of the markers according to the invention. Thus, the biological sample can be selected from the group of cells from a lung tumor, breast tumor, kidney tumor, liver tumor, head and neck tumor, prostate tumor, pancreatic tumor, stomach tumor, colon tumor, thyroid tumor, esophageal tumor, and brain tumor, whole blood, serum, plasma, urine, lymph fluid, brain liquor, tissue samples, and prepared tissue samples, such as histological slides. Preferred is a method according to the invention, wherein said sample is taken from the patient during surgery or is a serum sample.

The finding that macroH2A1.1 is enriched in rodent lung adenomas known to have a high number of senescent cells, but is downregulated or absent in lung carcinomas that have overcome senescence and display uncontrolled proliferation, is also consistent with these conclusions, underlining the idea of senescence as an antitumor mechanism. Thus, in this preferred aspect of the method according to the present invention, the expression can be used to generally monitor the amount or ratio of senescent cells, in particular of cancer cells, in the tumor based on the expression of the marker macroH2A1.1. The amount or ratio of senescent cells is an indicator for the growth of tumor cells is used in order to monitor the success or effect of an anti-tumor therapy, such as chemotherapy or irradiation, or to see whether the number of cancer cells is increasing, as an indication for a relapse of the patient. Other senescence markers can be measured, too, and methods for measuring or detecting said markers are known to the person of skill and as described herein for the markers macroH2A1.1 and/or macroH2A2.

In the context of the methods according to the present invention, any method known to the person of skill that allows for measuring the expression of a marker can be used. The choice of the method will depend on whether the expression is measured at the nucleic acid and/or protein level, and on the sample that is to be analyzed. Preferred is a method according to the present invention, wherein said detecting the expression comprises polymerase chain reaction (PCR), i.e. nucleic acid amplification, in particular rtPCR, or immunohistochemical staining (i.e. on the protein level), in particular using a macroH2A1.1-specifc or macroH2A2-specifc antibody, as described herein, and/or mRNA analysis.

Histones are often assumed to be present at similar levels in distinct cell types. The heterochromatic histone variant macroH2A is unusual in several molecular and cellular features. At the structural level, it bears a large C-terminal extension, the macro domain, a globular module of approx. 25 kDa, which in the case of macroH2A1.1 binds ADP-ribose with high specificity (Kustatscher el al., 2005). Further, there are two genes for macro-H2A, conserved in all vertebrates, and a second splice variant for macroH2A1, known as macroH2A1.2, which cannot bind small molecules. Here, the inventors have developed polyclonal and monoclonal antibodies against all macroH2A isoforms (macroH2A1.1, macroH2A1.2 and macroH2A2) and used these antibodies to study the cellular localization and abundance of macroH2A histones in cultured cells and in vivo. The inventors have found that macroH2A is upregulated in primary fibroblasts that have undergone replicative senescence. In addition, there is a strong negative correlation between the expression of macroH2A1.1 and Ki-67 in human cancer. Tumors undergoing rapid cell division (marked by high expression of the proliferation marker Ki-67) express low levels of macroH2A1.1, whereas tumors with low mitotic index are enriched for the histone. Both findings argue that macroH2A1.1 is correlated to the degree of differentiation of cells and marks cells that have exited the cell cycle.

The finding that macroH2A1.1 is enriched in rodent lung adenomas known to have a high number of senescent cells, but is downregulated or absent in lung carcinomas that have overcome senescence and display uncontrolled proliferation, is also consistent with these conclusions, underlining the idea of senescence as an antitumor mechanism. Significantly, the inventors find that macroH2A1.1 levels are a good predictor for the risk of tumor recurrence in lung cancer. In the defined number of patients the inventors could analyze, it proved to be statistically superior to the correlation with the common proliferation marker, Ki-67 (P=0.0058 for macroH2A1.1 and P=0.07983 for Ki-67).

Currently available patient information for the breast cancer tissue array is insufficient to assess correlations between macroH2A1.1 levels and clinical outcome. On the basis of the data from lung tumors, macroH2A1.1 appears to be a useful prognostic biomarker also in breast cancer. Further testing will reveal its usefulness in other cancers.

The mechanisms underpinning macroH2A1.1 gene product upregulation during cellular senescence and its role in carcinogenesis remain to be elucidated. Preliminary evidence suggests that mRNA levels do not alter during senescence, suggesting that post-transcriptional processes may account for the upregulation of macroH2A1.1.

The statistically supported observation of macroH2A1.1 expression with disease-free survival opens the prospect of an improved stratification of recurrence risk in cancer and particularly lung cancer patients and should be repeated with a bigger patient population. Future studies will provide even more insight into whether macroH2A1.1 upregulation is a general feature of senescent cells in human tumors and whether high macroH2A1.1 staining generally reflects the degree of cellular differentiation in tumor cells. The inventive new tool will improve the identification of high-risk cancer and particularly breast or lung cancer patients, thus allowing for better targeting of therapeutic strategies in cancers with difficult prognosis.

The invention shall now be further described in the following examples with reference to the accompanying Figures, nevertheless, without being limited thereto. In the Figures,

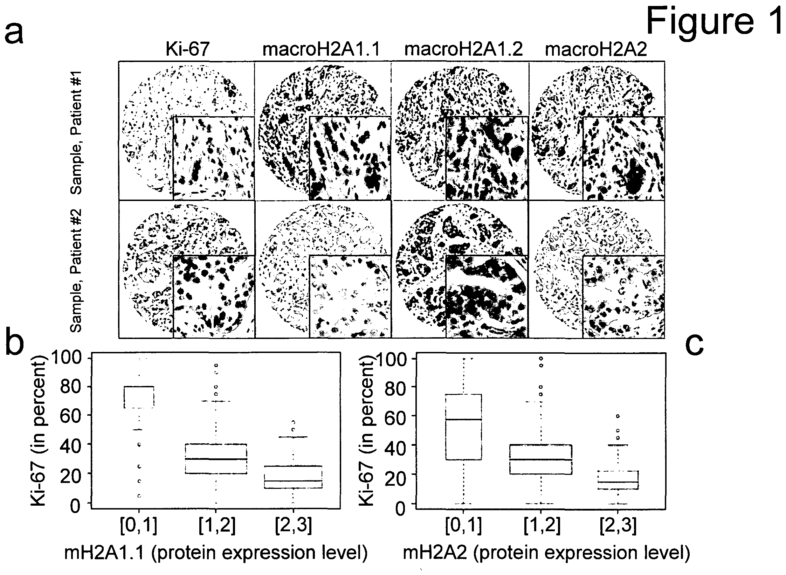

Figure 1 shows that an expression of macroH2A isoforms correlates with proliferation. (a) Proliferation index and levels of histone variants in a set of 260 breast cancer samples are determined by immunohistochemistry with antibodies against the proliferation marker Ki-67, macroH1A1.1, macroH2A1.2, and macroH2A2. Ki-67 was scored as the percentage of positively stained tumor cells for all tumor cells within a core. Expression levels of macroH2A1.1 were determined in a semi-quantitative way (scoring: 0-1, negative/weak staining; 1-2, moderate staining; 2-3, strong nuclear positivity). (b) Boxplots showing the decreasing expression of macroH2A1.1 with increasing proliferation index (P<0.001). (c) The same correlation is observed far macroH2A2. MacroH2A1.2 is expressed ubiquitously at a high level in these samples.

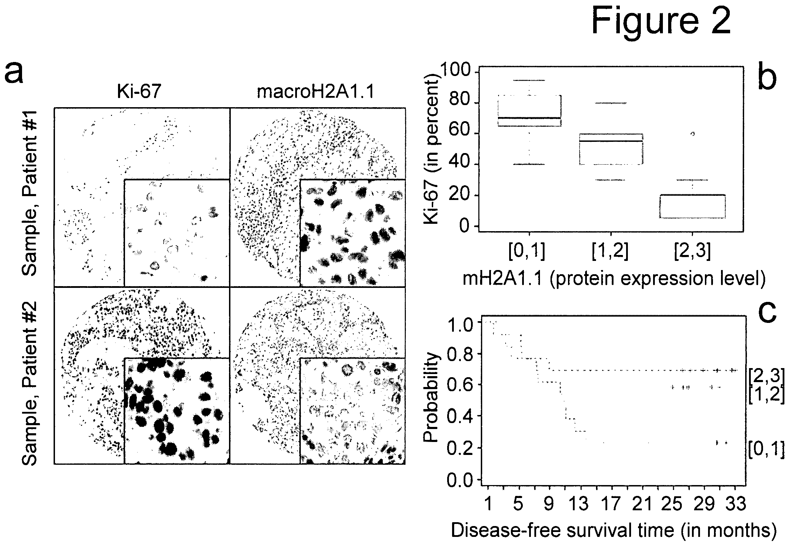

Figure 2 shows that macroH2A1.1 expression predicts cancer, and preferably lung cancer, recurrence. (a) Proliferation indices of lung tumors are determined by immunohistochemistry with antibodies against Ki-67. (b) Expression levels of macroH2A1.1 decrease with increasing proliferation indices *(P<0.001).* (c) Expression levels show a significant correlation with disease-free survival in lung tumor samples *(P*=*0.0058).*

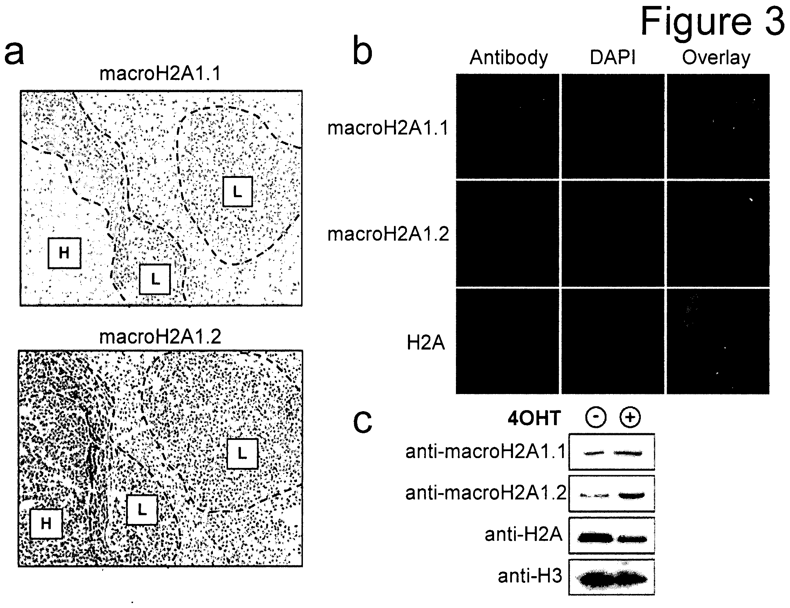

Figure 3 shows that macroH2A isoforms are upregulated during senescence. (a) Expression of macroH2A1 isoforms in the lungs of K-Ras+ / LSLC12Vgeo knock-in mice. Immunohistochemical detection of macroH2A1.1 (upper panel) and macroH2A1.2 levels (lower panel) was analyzed in consecutive sections of paraffin-embetted lung tissue containing both low-grade adenomas (L) and high-grade adenocarcinomas (H). MacroH2A1.1 expression is enriched during senescence in low-grade adenomas (b) Immunostaining of proliferating (small nuclei) and senescent (enlarged nuclei) W138 primary fibroblasts. MacroH2A1.1 and macroH2A1.2 localize to senescence-associated heterochromatic foci (SAHF) and are expressed at higher levels in senescent cells. In contrast, canonical histone H2A is not changed by senescence. (e) Western blot of macroH2A1 upregulation during senescence ITM cells are primary fibroblasts engineered to undergo simultaneous oncogene-induced senescence on addition of the tamoxifen analog 4OHT (Collado at al., 2005). The inventors consistently observe elevated levels of macroH2A1 in senescent cells. Protein levels of canonical histones H2A and H3 are slightly reduced in senescent cells.

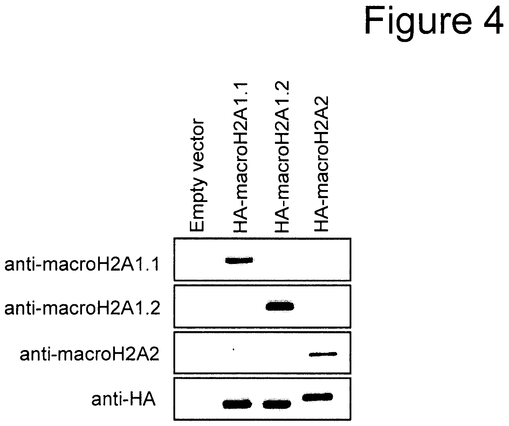

Figure 4 shows a Western blot showing the specificity of affinity-purified antibodies for the three macroH2A isoforms. HA-tagged macroH2A isoforms were expressed in HEK293 cells and whole-cell extracts were loaded for Western blot analysis.

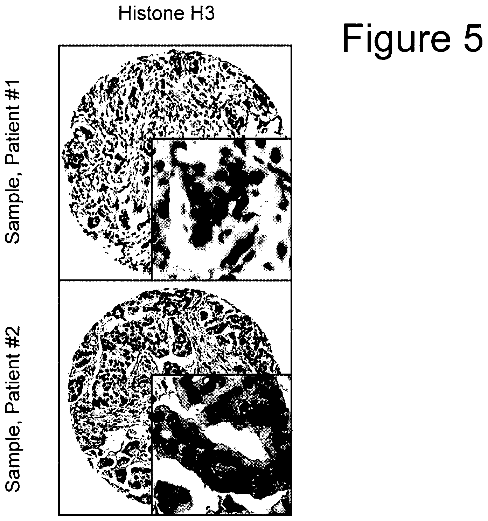

Figure 5 shows the expression of macroH2A isoforms in a set of 260 breast cancer samples. (a) Proliferation index and levels of histone variants are determined by immunohistochemistry with antibodies against Ki-67, macroH2A1.1, macroH2A1.2, macroH2A2. (b) Boxplots showing that the expression level of macroH2A1.1 decreases with increasing proliferation index (P<0.001). (c) The same correlation is observed for the macroH2A2 isoform. Note that macroH2A1.2 is expressed ubiquitiously at a high level in these samples.

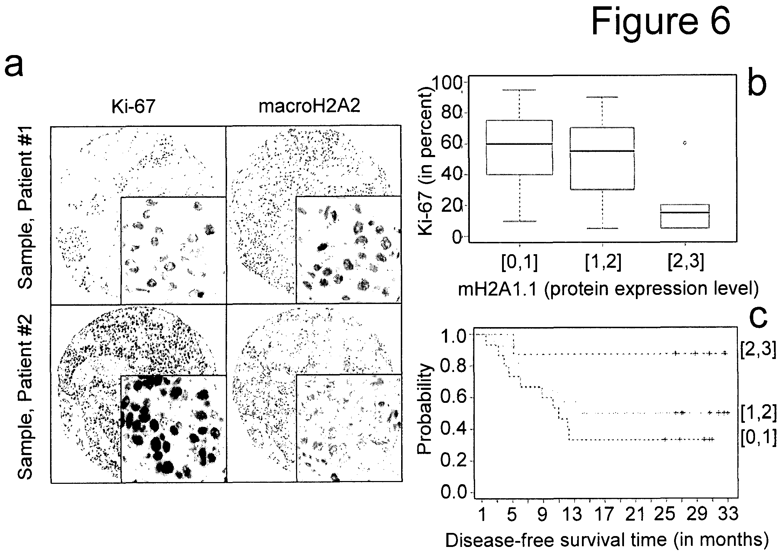

Figure 6 shows expression levels of mH2A2 in lung cancer. (a) Proliferation index and expression levels of histone variants are determined by immunohistochemical detection of Ki-67 and macroH2A2. (b) Expression levels of macroH2A2 decrease with increasing proliferation (P<0.001). (c) Expression levels of macroH2A2 correlate with tumor recurrence (P=0.04146).

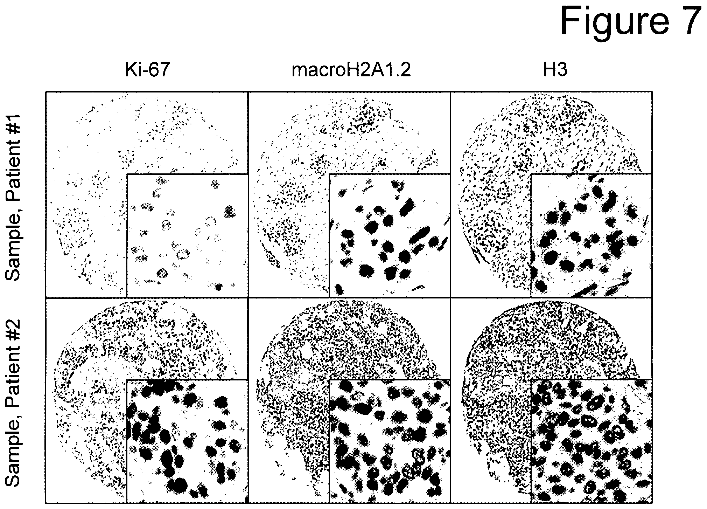

Figure 7 shows that macroH2A1.2 is expressed at high levels in most lung tumors. Proliferation index, expression of histone variants and core histones are determined by immunohistochemical detection of Ki-67, macroH2A1.2 and H3. There is no correlation between mH2A1.2 and Ki-67 expression, histone H3 is strongly expressed in all cells.

SEQ ID No. 1 to SEQ ID No. 8 show the sequences of oligonucleotides used in the Examples, below.

### Examples

### Materials and methods

### Histone and mRNA extraction, Western blotting and real-time PCR

The generation of ITM cells expressing the switchable MEK1-ER fusion protein was described earlier. After incubation with or without 40HT, cells were harvested and aliquoted. One cell aliquot was resuspended in 0.4 M HCl, incubated for 4 h to extract histone proteins, centrifuged and dialyzed into water. RNA was extracted using the RNeasy Mini Prep kit (Qi-agen). Proteins were precipitated with acetone from the flowthrough of the RNAeasy spin columns. Protein extracts were normalized by Bradford assay before loading. Western blots were carried out using isoform-specific macroH2A antibodies, as well as commercial antibodies against H2A (Abcam, ab15653; 1:3000) and H3 (Abcam; ab1791; 1:30000). First-strand cDNA synthesis was carried out using the Superscript II reverse transcriptase (Invitrogen). The cDNAs were subsequently analyzed by Real-time PCR (Applied Biosystems) using the following oligos:
mH2A1.1 AAGTTGTACAGGCTGACATTGC (SEQ ID No. 1);
GTTCTTTTTCCGGAGTTCCA (SEQ ID No. 2),
mH2A1.2 CTTTGAGGTGGAGGCCATAA (SEQ ID No. 3);
CACAAACTCCTTGCCACCTT (SEQ ID No. 4),
mH2A2 GGACGTCCAAAAAGTCCAAA (SEQ ID No. 5);
GCTTCTGTCCCAGAACAAGG (SEQ ID No. 6),
and GAPDH TGGGTGTGAACCATGAGAAGTA (SEQ ID No. 7);
CCTTCCACGATACCAAAGTTGT (SEQ ID No. 8).

Samples were normalized to GAPDH, which the inventors previously identified as a reliable control for these experiments in test runs.

### Generation of antibodies

MacroH2A1.1 (amino acids 198 - 226) and macroH2A1.2 (aa 198 - 228) were expressed and purified as GST-fusion proteins. The macro domain of macroH2A2 (aa 162 - 372) was purified as a (His)6-tag fusion protein. Antigens were injected into rabbits (Eurogentec). Antibodies were recovered from rabbit serum using HiTrap Protein-G HP columns (GE Healthcare Life Sciences) according to the manufacturer's instructions. Recombinant, (His)6-tagged macro domains of the three macroH2A isoforms were coupled to HiTrap NHS-activated HP (GE Healthcare). These columns were subsequently used for the affinity purification of isoform-specific antibodies.

### Breast cancer tissue microarrays

Breast cancer TMAs were provided by the National Center of Tumor Diseases (NCT), Heidelberg, Germany. They contained formalin-fixed and paraffin-embedded samples of 260 patients, with two samples per patient on each slide, distributed to different blocks. All breast cancer patients were female, median age of 58 .years (range: 30-88 years). Clinical data associated with these patients were insufficient for further analysis.

### Lung cancer patients and lung tissue microarrays

Lung cancer TMAs were provided by the Thoraxklinik Heidelberg, Germany, and the NCT. The slides contained samples of 41 patients with NSCLC that were found to be resectable and underwent surgery between March and December 2004 (23 with adenoma and 18 with squamous cell carcinoma).

Samples of these patients collected during surgery were formalin-fixed and paraffin-embedded and used to construct a TMA, which also included control samples. For quality reasons samples of two patients could not be analyzed, such that 39 patient samples were included in the analysis. See Table 1 for the clinical characteristics of the study population. The collection and use of the human materials was approved by the local ethics committee (ethical vote # 270/2001, updated 2005). Patients gave informed consent.

**Table 1: Characteristics of the non-small cell lung cancer study population.**

| **Characteristic** | **Median (Range)/Frequency** |
|---|---|
| Sex (male/female) | 31/8 |
| Age at diagnosis (years) | 64 (43-80) |
| ECOG performance status (0/1/2) | 28/11/0 |
| Tumor cell type (AC/SCC) | 22/17 |
| Type of operation (LO-BI-PN) | 26/2/11 |
| Resection status (R0/R1/R2) | 32/5/2 |
| Stage of disease (post-op) (Ia/Ib/IIa/IIb/IIIa/IIIb/IV) | 1/11/0/4/13/6/4 |
| Tumor recurrence within a year (y/n) | 18/21 |
| AC = adenocarcinoma; SCC = squamous cell carcinoma; BI = bilobectomy; LO = lobectomy; PN = pneumonectomy | |

Of the R2-resected patients 1 recurred (distant metastasis), 1 lived without recurrence; of the R1-resected patients 3 recurred (2 local recurrence, 1 distant metastasis), 2 lived without recurrence; of the R0-resected patients 15 recurred, 17 lived without recurrence.

### Immunohistochemistry

Immunohistochemical staining for macroH2A1.1, macroH2A1.2, macroH2A2, Ki-67 (Dako-Cytomation, monoclonal mouse anti-human Ki-67, clone MIB-1, 1:100) and histone H3 (Abcam; ab1791; 1:1000 dilution) was performed using successive TMA slides. Slides were scanned at the Department of Pathology (Heidelberg University) using the Aperio Scanscope System and ImageScope software.

Expression levels of macroH2A were assessed semi-quantitatively using the scoring: 0-1, negative/weak staining; 1-2, moderate staining; 2-3, strong nuclear positivity. Proliferation indexes were determined with antibodies against the proliferation marker Ki-67 and scored as the percentage of positively stained tumors comparison with all the tumor cells within a core. All slides were evaluated and scored blindly, and repeated twice.

For the analysis of the macroH2A isoforms in mouse tumors, lungs from activated 7-9 months old K-Ras P/G12Vgeo mice (Guerra et al., 2003) were dissected, fixed in 10%-buffered formalin (Sigma) and embedded in paraffin. Consecutive 3- to 5-mm-thick sections were stained with hematoxylin and eosin, and processed for immunohistochemical staining using macroH2A1.1 and macroH2A1.2 antibodies (1:75 dilution). Grading of lung lesions was done according to the latest morphological accepted criteria for mouse lung cancer (Jackson et al., 2005) by an expert pathologist. Grade 1-3 neoplasms were classified as adenomas (benign lesions) and grade 4-5 as adenocarcinomas (malignant lesions).

### Statistical analysis

The relationship between Ki-67 and mH2A1.1 was assessed using a conditional linear association test stratified by array (Hothorn et at., 2006). The dependence of disease-free survival was established by a conditional log-rank test, also stratified by array (Hothorn et al., 2006).

There are three gene products for the two human macroH2A genes, all containing an N-terminal H2Alike fold and a C-terminal macro domain. MacroH2A1.1 and macroH2A1.2 are produced through alternative splicing of the H2AFY gene product, whereas macroH2A2 is encoded by a second gene, H2AFY2. In vitro, the macro domain of macroH2A1.1, but not the other isoforms, binds ADP-ribose and related NAD metabolites (Kustatscher et al., 2005). The inventors generated specific antibodies to detect and distinguish the different macroH2A isoforms (Figure 4).

It has been suggested that macroH2A1.1 expression may be restricted to differentiated, non-proliferative tissues (Pehrson et al., 1997). In order to analyze any correlations between macroH2A1.1 protein expression and cell proliferation; the inventors performed immunohistochemical stainings on tissue microarrays (TMAs) containing breast cancer-samples of 260 patients (Figure 1). The inventors assessed the expression levels of macroH2A and the proliferation marker Ki-67 in all tumor cells within a core. Histone H3 staining served as a positive control and showed strong nuclear staining in every cell within the cores (Figure 5). The inventors assessed the relationship between Ki-67 and macroH2A1.1 expression using a conditional linear association test stratified by array. The inventors find that macroH2A1.1 expression is highly correlated to Ki-67 levels (P<0.001), showing a decrease in macroH2A1.1 with the increase of Ki-67 positive cancer cells. A similar relationship is observed for mH2A2 (P<0.001), whereas macroH2A1.2 is expressed ubiquitously in these tumors (Figure 1). The latter could be in part due to a higher affinity of the antibody against macroH2A1.2, which might make it difficult to compare subtle changes in the expression levels. However, there clearly are tissues in which the inventors can see and discriminate highly varying expression levels for macroH2A1.2 (such as skeletal muscle).

These assays establish that the expression of the splice variant macroH2A1.1 is limited to tumors showing lower proliferation indices.

In order to test how cell proliferation and macroH2A1.1 levels correlate in a set of human tumors with complete clinical parameters, the inventors scored samples from 39 NSCLC patients, which were found resectable and underwent surgery (22 adenocarcinomas and 17 squamous cell carcinomas; Table 1). Within 1 year after surgery, tumors recurred in 18 patients, whereas 21 patients remained recurrence-free. Similar to what the inventors observe in breast cancer and other cancer arrays, statistical analysis of the lung tumor array reveals that macroH2A1.1 expression is strongly correlated with Ki-67 levels (P<0.001) and decreases with the increase of Ki-67 positive cancer cells (Figure 2a and b). This substantiates the evidence that macroH2A1.1 is a marker for cells that have exited the cell cycle.

To further test this notion, the inventors analyzed immunohistochemical sections from a mouse model of tumor senescence induced by K-RasGI2V (Guerra el al., 2003). The stainings reveal higher macroH2A1.1 expression in premalignant adenoma lesions, in which hallmark is an abundance of senescent cells (Braig er al., 2005; Chen et al., 2005; Collado et al., 2005), in comparison to the corresponding malignant adenocarcinomas (Figure 3a). In contrast, the splice variant macroH2A1.2 is expressed at similar levels in adenomas and adenocarcinomas. This expression pattern defines macroH2A1.1 as an oncogene-induced senescence marker.

To identify whether there is a dependence of disease-free survival (recurrence) with macroH2A1.1 expression, the inventors carried out a conditional log-rank test, stratified by array (Hothorn et al., 2006).

Interestingly, it reveals a significant correlation between macroH2A1.1 expression and disease-free survival (P=0.0058). Patients with low macroH2A1.1 levels in lung tumor samples recur more likely than those with strong nuclear macroH2A1.1 staining (Figure 2c). A similar, but weaker, correlation is also observed for the expression of macroH2A2 (P=0.04146; Figure 6). In contrast; macroH2A1.2, which is ubiquitously expressed (Figure 7). shows no prognostic correlation to tumor recurrence. It is noted that the expression of the common proliferation marker Ki-67 does not show a significant correlation with disease-free survival (P=0.07983). Thus, the inventors' immunostaining and clinical data identify histone macroH2A1.1 as a novel, superior and potentially useful biomarker of lung cancer recurrence.

To further characterize the heterochromatic histone macroH2A during senescence, the inventors performed immuno-fluorescent stainings of primary fibroblasts and observe that macroH2A1 isoforms are upregulated during replicative senescence. MacroH2A1.1 is expressed at higher levels in pre-senescent and senescent cells, as well as localizing to senescence-associated heterochromatic foci (SAHF) in senescent cells (Figure 3b). Similarly, macroH2A1.2 is also upregulated, in contrast to control proteins, such as the canonical histone H2A. Further, the enrichment of macroH2A in SAHF is not a mere consequence of the condensed nature of SAHF chromatin, as cellular macroH2A enrichment and SAHF incorporation follow slower kinetics than the formation of DAPI-enriched heterochromatic structures, confirming earlier reports (Zhang et al., 2005). This finding is supported by Western blots on human normal diploid fibroblasts IMR90, expressing hTERT and MEK:ER (ITM cells; Collado et al., 2005). This system allows for switchable activation of MEK kinase, a downstream effector of Ras-induced senescence. The results show upregulation of macroH2A1.1 and macroH2Al.I histone protein in senescent cells (Figure 3c). In contrast, canonical histone H2A seems to be mildly downregulated on the induction of senescence.

### Literature as cited

Braig M. Lee S, Loddenkemper C. Rudolph C, Peters AH, Schlegelberger B et al. (2005). Oncogene induced senescence as an initial barrier in lymphoma development. Nature 436: 660-665.
Chen Z, Trotman LC, Shaffer D, Lin HK, Dotan ZA, Niki M et al. (2005). Crucial role of p53-dependent cellular senescence in suppression of Pten-deficient tumorigenesis. Nature 436: 725-730.
Collado M, Gil J, Efeyan A; Guerra C, Schuhmacher AJ, Barradas M et al. (2005). Tumour biology: senescence in premalignant tumours. Nature 436: 642.
Collado M, Serrano M. (2006). The power and the promise of oncogene-induced senescence markers. Nat Rev Cancer 6: 472-476.
D'Amico TA. (2008). Molecular biologic staging of lung cancer. Ann Thorac Surg 85: S737-S742.
Guerra C, Mijimolle N, Dhawahir A, Dubus P, Barradas M, Serrano M at al. (2003). Tumor induction by an endogenous K-ras oncogenes is highly dependent on cellular context. Cancer Cell 4: 111-120.
Hayflick L. (1965). The limited in vitro lifetime of human diploid cell strains. Exp Cell Res 37: 614-636.
Hothorn, T., Hornik, K., Van de Wiel, M.A. & Zeileis, A. A Lego system for conditional inference. American Statistician 60, 257-263 (2006).
Jackson EL, Olive KP, Tuveson DA, Bronson R, Crowley D, Brown M at al. (2005). The differential effects of mutant p53 alleles on advanced murine lung cancer. Cancer Res 65: 10280-10288.
Jemal A, Siegel R, Ward E, Murray T, Xu J, Thun MJ. (2007). Cancer statistics, 2007. CA Cancer J Clin 57: 4366.
Kustatscher G, Hothorn M. Pugieux C, Scheffiek K, Ladurner AG. (2005). Splicing regulates NAD metabolite binding to histone macroH2A. Nat Struct Mol Biol 12: 624-625.
Michaloglou C, Vredeveld LC, Soengas MS, Denoyelle C, Kuilman T, van der Horst CM et al. (2005). BRAFE600-associated senescence-like cell cycle arrest of human naevi. Nature 436: 720-744.
Narita M, Nuner S, Heard E, Narita M, Lin AW, Hearn SA at al. (2003). Rb-mediated heterochromatin formation and silencing of E2F target genes during cellular senescence. Cell 113: 703-716.
Pehrson JR. Costanzi C. Dharia C. (1997) Developmental and tissue expression patterns of histone macroH2A1 subtypes. J Cell Biochem 65: 107-113.
Pehrson JR, Fried VA. (1992). MacroH2A, a core histone containing a large nonhistone region. Science 257: 1398-1400.
Serrano M, Lin AW; McCurrach ME, Beach D, Lowe SW. (1997). Oncogenic ras provokes premature cell senescence associated with accumulation of p53 and p161NK4a. Cell 88: 593-602.
Zhang R; Poustovoitov MV, Ye X, Santos HA; Chen W; Daganzo SM at al. (2005). Formation of MacroH2A-containing senescence associated heterochromatin foci and senescence driven by ASF1a and HIRA. Dev. Cell 8: 19-30.
Zhu CQ, Shih W, Ling CH; Tsao MS. (2006). Immunohistochemical markers of prognosis in non-small cell lung cancer: a review and proposal for a multiphase approach to marker evaluation. J Clin Pathol 59: 790-800.

## Claims

1. An *in-vitro* method for diagnosing the risk of cancer recurrence in a mammalian patient, comprising detecting the expression of macroH2A1.1 and/or macroH2A2 in a biological sample obtained from said patient, wherein a low or reduced expression of said macroH2A1.1 and/or macroH2A2 is indicative for an increased risk of cancer recurrence in said patient.

2. The *in vitro* method according to claim 1, wherein said expression is normalized to the expression of macroH2A1.2 in said sample and/or a control sample.

3. The *in vitro* method according to claim 1 or claim 2, wherein a correlation between the expression of macroH2A1.1 or macroH2A2 and the disease-free survival is P < 0.05, preferably P < 0.01, and more preferably P < 0.006.

4. The *in vitro* method according to any of claims 1 to 3, wherein said cancer is selected from the group of solid cancer, lung cancer, breast cancer, kidney cancer, liver cancer, head and neck cancer, prostate cancer, pancreatic cancer, stomach cancer, colon cancer, thyroid cancer, esophageal cancer, and brain cancer.

5. The *in vitro* method according to any of claims 1 to 4, wherein said biological sample is selected from the group of cells from a lung tumor, breast tumor, kidney tumor, liver tumor, head and neck tumor, prostate tumor, pancreatic tumor, stomach tumor, colon tumor, thyroid tumor, esophageal tumor, and brain tumor, whole blood, serum, plasma, urine, lymph fluid, pleural fluid, and brain liquor.

6. The *in vitro* method according to any of claims 1 to 5, further comprising detecting the expression of the additional cancer marker Ki-67.

7. The *in vitro* method according to claim 6, wherein said expression of said macroH2A1.1 and/or macroH2A2 in said sample inversely correlates with the expression of Ki-67.

8. The *in vitro* method according to any of claims 1 to 7, further comprising a diagnosis of senescent cells, in particular of cancer cells, in the tumor based on the expression of macroH2A1.1.

9. The *in vitro* method according to any of claims 1 to 5, further comprising a prediction of said cancer recurrence based on said diagnosis.

10. The *in vitro* method according to any of claims 1 to 9, wherein said sample is taken from the patient during surgery.

11. The *in vitro* method according to any of claims 1 to 10, wherein said detecting the expression comprises PCR, in particular rtPCR, immunohistochemical staining, in particular using a macroH2A1.1-specific or macroH2A2-specific antibody, and/or mRNA analysis.

## Patentansprüche

1. Ein In-vitro-Verfahren zur Diagnose des Risikos einer Krebsrekurrenz in einem Säugetierpatienten, umfassend Nachweisen der Expression von macroH2A1.1 und / oder macroH2A2 in einer biologischen Probe, die von genanntem Patienten abgenommen wurde, wobei eine niedrige oder reduzierte Expression von genanntem macroH2A1.1 und/oder macroH2 ein erhöhtes Risiko einer Krebsrekurrenz in genanntem Patienten anzeigt.

2. Das In-vitro-Verfahren gemäß Anspruch 1, wobei genannte Expression an die Expression von macroH2A1.2 in genannter Probe und / oder einer Kontrollprobe normalisiert ist.

3. Das In-vitro-Verfahren gemäß Anspruch 1 oder Anspruch 2, wobei eine Korrelation zwischen der Expression von macroH2A1.1 oder macroH2A2 und dem erkrankungsfreien Überleben P <0,05, vorzugsweise P <0,01, und weiter bevorzugt P <0,006 ist.

4. Das In-vitro-Verfahren gemäß eines der Ansprüche 1 - 3, wobei genannter Krebs ausgesucht wird von der Gruppe solider Krebsarten, Lungenkrebs, Brustkrebs, Nierenkrebs, Leberkrebs, Kopf- und Nackenkrebs, Prostatakrebs, Pankreaskrebs, Magenkrebs, Darmkrebs, Thyroidkrebs, Krebs des Ösophagus, und Gehirnkrebs.

5. Das In-vitro-Verfahren gemäß eines der Ansprüche 1 - 4, wobei genannte biologische Probe ausgesucht wird aus der Gruppe von Zellen eines Lungentumors, Brusttumors, Nierentumors, Lebertumors, Kopf- und Nackentumors, Prostatatumors, Pankreastumors, Magentumors, Darmtumors, Thyroidtumors, Ösophagustumors, und Gehirntumors, Gesamtblut, Serum, Plasma, Urin, Lymphflüssigkeit, Pleuralflüssigkeit, und Gehirnflüssigkeit.

6. Das In-vitro-Verfahren gemäß eines der Ansprüche 1 - 5, weiter umfassend Nachweisen der Expression eines zusätzlichen Krebsmarkers Ki-67.

7. Das In-vitro-Verfahren gemäß Anspruch 6, wobei genannte Expression von genanntem macroH2A1.1 und / oder macroH2A2 in genannter Probe invasiv korreliert mit der Expression von Ki-67. 8.

8. Das In-vitro-Verfahren gemäß eines der Ansprüche 1 - 7, weiter umfassend eine Diagnose seneszenter Zellen, insbesondere von Krebszellen, in einem Tumor basierend auf der Expression von macroH2A1.1.

9. Das In-vitro-Verfahren gemäß eines der Ansprüche 1 - 5, weiter umfassend ein Vorhersagen genannter Krebsrekurrenz basierend auf genannter Diagnose.

10. Das In-vitro-Verfahren gemäß eines der Ansprüche 1 - 9, wobei genannte Probe von genanntem Patienten während einer Operation entnommen wurde.

11. Das In-vitro-Verfahren gemäß eines der Ansprüche 1 - 10, wobei genanntes Nachweisen der Expression eine PCR umfasst, insbesondere eine rtPCR, immunhistochemisches Färben, insbesondere unter Verwendung eines macroH2A1.1-spezifischen oder macroH2A2-spezifischen Antikörpers, und / oder mRNA-Analyse umfasst.

## Revendications

1. Procédé *in vitro* pour diagnostiquer le risque de récurrence de cancer chez un patient mammifère, comprenant la détection de l'expression de macroH2A1.1 et/ou de macroH2A2 dans un échantillon biologique obtenu à partir dudit patient, dans lequel une expression faible ou réduite desdits macroH2A1.1 et/ou macroH2A2 indique un risque accru de récurrence de cancer chez ledit patient.

2. Procédé *in vitro* selon la revendication 1, dans lequel ladite expression est normalisée par rapport à l'expression de macroH2A1.2 dans ledit échantillon et/ou un échantillon témoin.

3. Procédé *in vitro* selon la revendication 1 ou la revendication 2, dans lequel une corrélation entre l'expression de macroH2A1.1 ou de macroH2A2 et la survie sans maladie est P < 0,05, de préférence P < 0,01, et de façon davantage préférée P < 0,006.

4. Procédé *in vitro* selon l'une quelconque des revendications 1 à 3, dans lequel ledit cancer est choisi dans le groupe d'un cancer solide, un cancer du poumon, un cancer du sein, un cancer du rein, un cancer du foie, un cancer de la tête et du cou, un cancer de la prostate, un cancer pancréatique, un cancer de l'estomac, un cancer du côlon, un cancer de la thyroïde, un cancer oesophagien, et un cancer du cerveau.

5. Procédé *in vitro* selon l'une quelconque des revendications 1 à 4, dans lequel ledit échantillon biologique est choisi dans le groupe de cellules provenant d'une tumeur du poumon, une tumeur du sein, une tumeur du rein, une tumeur du foie, une tumeur de la tête et du cou, une tumeur de la prostate, une tumeur pancréatique, une tumeur de l'estomac, une tumeur du côlon, une tumeur de la thyroïde, une tumeur oesophagienne, et une tumeur du cerveau, de sang total, de sérum, de plasma, d'urine, de liquide lymphatique, de liquide pleural, et de liqueur cérébrale.

6. Procédé *in vitro* selon l'une quelconque des revendications 1 à 5, comprenant en outre la détection de l'expression du marqueur cancéreux supplémentaire Ki-67.

7. Procédé *in vitro* selon la revendication 6, dans lequel ladite expression desdits macroH2A1.1 et/ou macroH2A2 dans ledit échantillon est en corrélation inverse avec l'expression de Ki-67.

8. Procédé *in vitro* selon l'une quelconque des revendications 1 à 7, comprenant en outre un diagnostic de cellules sénescentes, en particulier de cellules cancéreuses, dans la tumeur en se basant sur l'expression de macroH2A1.1.

9. Procédé *in vitro* selon l'une quelconque des revendications 1 à 5, comprenant en outre une prédiction de ladite récurrence de cancer en se basant sur ledit diagnostic.

10. Procédé *in vitro* selon l'une quelconque des revendications 1 à 9, dans lequel ledit échantillon est prélevé chez le patient durant une chirurgie.

11. Procédé *in vitro* selon l'une quelconque des revendications 1 à 10, dans lequel ladite détection de l'expression comprend une PCR, en particulier une rtPCR, une coloration immunocytochimique, en particulier en utilisant un anticorps spécifique de macroH2A1.1 ou spécifique de macroH2A2, et/ou une analyse d'ARNm.


### SEQUENCE LISTING

<110> European Molecular Biology Laboratory (EMBL)
<110> Thoraxklinik-Heidelberg gGmbH
<120> Diagnostic method for predicting the risk of cancer recurrence based on Histone macroH2A isoforms
<130> E31352PCT
<150> EP09008679.4
   <151> 2009-07-02
<160> 8
<170> PatentIn version 3.5
<210> 1
   <211> 22
   <212> DNA
   <213> Homo sapiens
<400> 1
   aagttgtaca ggctgacatt gc 22
<210> 2
   <211> 20
   <212> DNA
   <213> Homo sapiens
<400> 2
   gttctttttc cggagttcca 20
<210> 3
   <211> 20
   <212> DNA
   <213> Homo sapiens
<400> 3
   ctttgaggtg gaggccataa 20
<210> 4
   <211> 20
   <212> DNA
   <213> Homo sapiens
<400> 4
   cacaaactcc ttgccacctt 20
<210> 5
   <211> 20
   <212> DNA
   <213> Homo sapiens
<400> 5
   ggacgtccaa aaagtccaaa 20
<210> 6
   <211> 20
   <212> DNA
   <213> Homo sapiens
<400> 6
   gcttctgtcc cagaacaagg 20
<210> 7
   <211> 22
   <212> DNA
   <213> Homo sapiens
<400> 7
   tgggtgtgaa ccatgagaag ta 22
<210> 8
   <211> 22
   <212> DNA
   <213> Homo sapiens
<400> 8
   ccttccacga taccaaagtt gt 22